# FASCICULE DE BREVET EUROPEEN

(11) **EP 1 322 284 B1**
(45) Date de publication et mention de la délivrance du brevet: **31.05.2006**
(21) Numéro de dépôt: 01974402.8
(22) Date de dépôt: 27.09.2001
(51) Int. Cl.: A61K 8/64, A61Q 19/00

(54) **PROCEDE D'OBTENTION D'UN PRINCIPE ACTIF A EFFET TENSEUR IMMEDIAT DE LA PEAU**
HERSTELLUNGSVERFAHREN VON EINEM WIRKSTOFF MIT AKUT-HAUTSPANNUNGSEFFEKT
METHOD FOR OBTAINING AN ACTIVE PRINCIPLE WITH IMMEDIATE SKIN-TIGHTENING EFFECT AND RESULTING ACTIVE PRINCIPLE

(30) Priorité: 02.10.2000 FR 0012561
(43) Date de publication de la demande: 02.07.2003
(73) Titulaire: Société Industrielle Limousine d'Application Biologique (SILAB), 19130 Objat (FR)
(72) Inventeur: PAUFIQUE, Jean-Jacques, F-19130 Objat (FR)
(74) Mandataire: Fantin, Laurent
(86) Numéro de dépôt international: PCT/FR2001/002995
(87) Numéro de publication internationale: WO 2002/028360

(56) Documents cités:
- EP-A- 0 186 025
- FR-A- 2 762 211
- FR-A- 2 778 565

## Description

La présente invention a pour objet un principe actif à effet tenseur immédiat de la peau, réalisé à partir de protéines polymérisées d'un tourteau d'amandes blanches. L'invention couvre aussi le procédé d'obtention et les compositions cosmétiques.

En cosmétique, de nombreux travaux sont engagés pour agir sur la peau en sorte d'obtenir un effet tenseur. C'est ainsi que l'on traite les rides et les faiblesses du derme en certains endroits plus fragiles afin de lutter contre les relâchements.

Il existe des traitements qui se prolongent à long terme qui agissent en profondeur. De tels traitements permettent de compenser les effets de l'âge au niveau cellulaire.

Par contre, les consommateurs sont demandeurs de produits permettant de disposer d'un effet liftant et lissant immédiat. Dans ce cas, c'est l'instantanéité du résultat qui est recherché.

On sait que le fait d'agir sur la peau et de provoquer une tension de cette peau, au moins superficiellement, conduit à des résultats tout à fait visibles car l'état de surface de la peau est amélioré.

Ainsi le teint lui-même s'en ressent puisque la lumière est renvoyée différemment par rapport à une peau présentant des relâchements.

L'art antérieur a déjà proposé des solutions pour lutter contre les agressions de la peau notamment en augmentant l'épaisseur du stratum corneum avec un principe actif issu d'un procédé comprenant un broyage de graines de lupin blanc doux, une solubilisation en phase aqueuse, en hydrolysant les protéines en présence d'une protéase. Ce procédé fait l'objet de la demande de brevet français FR-2.778.565.

La demande de brevet français FR-2.762.211, décrit un principe actif extrait de graines d'Hibiscus esculentus.

La demande de brevet européen EP-0.186.025 décrit une préparation cosmétique obtenue à partir d'un hydrolysat protéinique d'amandes.

Afin de lutter contre une perte de régularité de la surface de la peau, la présente invention propose un principe actif qui assure un effet tenseur immédiat.

Pour obtenir le résultat visé, on cherche un principe actif riche en protéines de haut poids moléculaire, toujours soluble dans l'eau, ce qui est un paramètre très utile dans le domaine de la cosmétologie.

A cet effet, selon l'invention, le procédé d'obtention d'un principe actif à effet tenseur immédiat de la peau se caractérise en ce qu'il comprend les étapes suivantes :
- solubilisation en phase aqueuse d'un tourteau d'amandes blanches, par exemple de Prunus Amygdalus Dulcis, à raison de 50 à 500 g/l et plus particulièrement de 100 à 300 g/l,
- hydrolyse enzymatique modérée de la solution à pH acide et à une température comprise entre 40 et 80°C,
- séparation des phases soluble et insoluble et inactivation du milieu pour interrompre la réaction enzymatique,
- polymérisation des protéines par ajout d'un initiateur de polymérisation, et
- séparation des protéines polymérisées par filtration, décantation, centrifugation et concentration.

L'invention couvre aussi le principe actif obtenu par la mise en oeuvre de ce procédé ainsi que les compositions cosmétiques destinées à procurer un effet tenseur immédiat présentées sous toute forme galénique adaptée.

La présente invention est maintenant décrite suivant un mode de réalisation particulier, à travers le procédé d'obtention et les résultats expérimentaux obtenus.

Le tableau annexé sous forme de dessins est relatif aux résultats obtenus avec le principe actif selon la présente invention et permet de caractériser les effets. Il est également joint une courbe de résultats.

Le procédé consiste à solubiliser en phase aqueuse un tourteau d'amandes blanches à raison de 50 à 500 g/l et plus particulièrement à raison de 100 à 300 g/l.

On fait subir à cette solution une hydrolyse à pH acide, à une température comprise entre 40 et 80°C, en présence d'une enzyme, avec une action enzymatique modérée.

On procède à la séparation des phases soluble et insoluble et on inactive le milieu pour interrompre l'action enzymatique.

Une étape essentielle du procédé selon la présente invention est une polymérisation des protéines retenues. Cette étape est réalisée par ajout d'un initiateur de polymérisation, en l'occurrence au moins 1% d'un composé multifonctionnel comme un polychlorure d'acides, un polyanhydride d'acides, un polyisocyanate, un polythioisocyanate.

Une séparation par filtration, centrifugation ou décantation permet de ne retenir que la fraction lourde des protéines ainsi polymérisées, à savoir la fraction ayant une masse molaire supérieure à 600 Kdaltons.

Le principe actif selon la présente invention est caractérisé par les données suivantes :
1/ Le taux de matière sèche est supérieur à 50 g/l, compris entre 50 et 500 g/l plus particulièrement entre 50 et 250 g/l.
   Ce taux est obtenu par passage à l'étuve à 105°C jusqu'à obtention d'un poids constant.
2/ Le pH est compris entre 2,0 et 10,0, notamment entre 4,0 et 10,0, plus particulièrement entre 7 et 9.
   Cette valeur est obtenue par la méthode potentiométrique, à température ambiante.
3/ Le taux de protéines par rapport aux matières sèches est supérieur à 70%.
4/ Profil chromatographique :
   La masse molaire des différentes espèces moléculaires est estimée par filtration moléculaire F.P.L.C (Fast Protein Liquid Chromatography).
   La colonne de filtration, du type commercialisée sous la marque "superdex 75" est étalonnée avec des marqueurs de masse moléculaire connues tels que :
   - cytochrome *C* : 12 500 daltons,
   - albumine bovine : 66 000 daltons,
   - alcool déshydrogénase : 150 000 daltons.

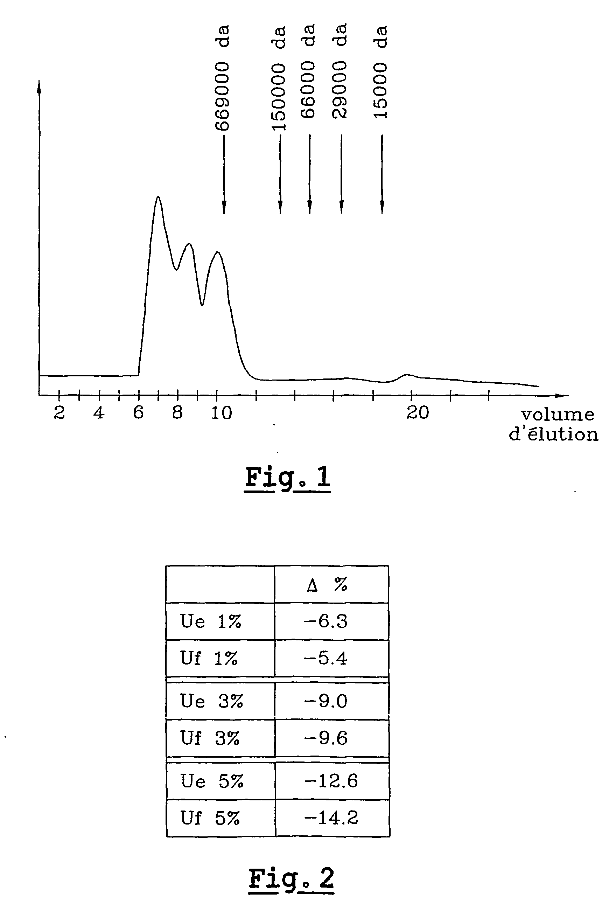

   La détection des composants élués est effectuée dans l'ultra-violet à 280 nm.
   On obtient les résultats indiqués dans le tableau de la figure 1 qui complètent les caractéristiques du principe actif obtenu.
5/ Détermination des poids moléculaires
   Cette détermination est réalisée par électrophorèse SDS-Page avec un gel à 10% polyacrylamide/bisacrylamide présentant un rapport de 36/1.
   On détecte deux fractions A et B :
   Fraction A : Présence de protéines de forme dimères avec des poids moléculaires de 30 et 51 Kdaltons,
   Fraction B: Présence de protéines de très haut poids moléculaire supérieur à 600 Kdaltons.

   La répartition des deux fractions est sensiblement 50/50.
6/ Autres paramètres:
   Le principe actif obtenu est stable pour des pH supérieurs à 5, des températures inférieures à 50°C en présence d'éthanol.

Afin de caractériser encore l'effet tenseur obtenu grâce à ce principe actif par exemple mis en émulsion, ceci sur des volontaires, on utilise comme moyen de contrôle un appareil du commerce connu sous la dénomination "Cutomètre" (Courage et Khazaka).

Un tel moyen de contrôle comprend une sonde avec un orifice. Cette sonde est appliquée sur la peau et une dépression constante est maintenue dans cette sonde.

On mesure la profondeur de pénétration de la peau dans cette sonde à travers l'orifice, sous l'effet de l'aspiration.

La peau, soumise à des dépressions, se fatigue plus ou moins vite et les temps de réponse comme les amplitudes permettent de déterminer notamment une déformation instantanée *Ue* dite composante élastique, une extensibilité *Uf.*

On réalise ainsi sur des volontaires une première série d'applications de l'émulsion contenant le principe actif selon l'invention contre placebo sur une zone déterminée, par exemple le visage, les bras ou les cuisses.

Une nouvelle série de mesures, identique à la première, aux mêmes endroits et avec les mêmes réglages de l'appareil de mesure, est conduite après une durée de 2 heures en sorte de déterminer l'effet.

Le tableau de la figure 2 récapitule ces résultats avec des pourcentages croissants de principe actif.

Lorsque la déformation instantanée *Ue* diminue, la peau est moins souple donc plus tendue et si *Uf* diminue la peau est moins extensible donc plus tendue également.

On constate que 2 heures après l'application isolée, on obtient un effet tenseur dose-dépendant très intéressant et statistiquement significatif et que cet effet est aussi bien perçu sur les différentes zones étudiées.

Le principe actif peut être associé à toute forme galénique : crème, gel, onguent, lotion, lait, émulsion ou solution, et à des excipients adaptés et connus de l'homme de l'art pour en permettre un meilleur usage.

On peut citer les exemples de composition suivants avec un principe actif ayant un taux de matière sèche de 121 g/l, un taux de protéines de 118 g/l et un pH de 7,8:
1/ Composition gel effet tenseur :
   - Montanov 202:3%
   - Lanol 99 : 2%
   - Lanol 1688 : 10%
   - Principe actif selon la présente invention : 3%
   - Sepigel 305 : 2%
   - Conservateur : 0,5%
   - Eau qsp : 100
2/ Lait corporel soin du corps amincissant et lissant :
   - Lanol 2681 : 2%
   - Lanol 99 :3%
   - Dc 345 : 5%
   - Glycerol : 2%
   - Sepigel 501 : 3%
   - Ethanol: 8%
   - Consrvateur : 0,5%
   - Actif amincissant : 3%
   - Principe actif selon l'invention : 3%
   - Eau qsp : 100

## Revendications

1. Procédé d'obtention d'un principe actif à effet tenseur immédiat de la peau, **caractérisé en ce qu'**il comprend les étapes suivantes :
- solubilisation en phase aqueuse d'un tourteau d'amandes blanches à raison de 50 à 500 g/l,
- hydrolyse enzymatique modérée à pH acide et à une température comprise entre 40 et 80°C,
- séparation des phases soluble et insoluble et inactivation du milieu pour interrompre la réaction enzymatique,
- polymérisation des protéines retenues par ajout d'un initiateur de polymérisation, et
- séparation des protéines polymérisées.

2. Procédé d'obtention d'un principe actif a effet tenseur immédiat de la peau selon la revendication 1, **caractérisé en ce que** la solubilisation est réalisée à raison de 100 à 300 g/l de tourteau d'amandes blanches.

3. Procédé d'obtention d'un principe actif a effet tenseur immédiat de la peau selon la revendication 1 ou 2, **caractérisé en ce que** le tourteau d'amandes blanches est un tourteau d'amandes de Prunus Amygdalus Dulcis.

4. Procédé d'obtention d'un principe actif à effet tenseur immédiat de la peau selon l'une quelconque des revendications précédentes, **caractérisé en ce que** l'initiateur de polymérisation comprend au moins 1% d'un composé multifonctionnel comme un polychlorure d'acides, un polyanhydride d'acides, un polyisocynnate, un polythioisocyanate.

5. Procédé d'obtention d'un principe actif à effet tenseur immédiat de la peau selon l'une quelconque des revendications précédentes, **caractérisé en ce que** la fraction de protéines polymérisée présente une masse moléculaire supérieure à 600 Kdaltons.

6. Principe actif obtenu par la mise en oeuvre du procédé défini selon l'une quelconque des revendications 1 à 5, **caractérisé en ce qu'** il présente les paramètres suivants :
- taux de matière sèche supérieur à 50g/l, de préférence compris entre 50et 500 g/l et plus particulièrement limité à la plage 50/250 g/l.
- pH compris entre 2,0 et 10,0, notamment 4,0 et 10,0, plus spécifiquement 7,0 et 9,0.
- taux de protéines par rapport aux matières sèches supérieur à 70%.
- répartition des poids moléculaires 50/50 de
* protéines de forme dimère de poids moléculaire compris entre 30 et 51 Kdaltons
* protéines de très haut poids moléculaire supérieur à 600 Kdaltons.

## Claims

1. A method of obtaining an active principle with immediate firming effect on the skin, **characterised in that** it comprises the following steps:
- solubilisation in aqueous phase of a cake of white almonds at the rate of 50 to 500 g/l,
- moderate enzymatic hydrolysis at an acidic pH and at a temperature of between 40° and 80°C,
- separation of the soluble and insoluble phases and inactivation of the medium in order to interrupt the enzymatic reaction,
- polymerisation of the retained proteins by the addition of a polymerisation initiator, and
- separation of the polymerised proteins.

2. A method of obtaining an active principle with an immediate firming effect on the skin according to claim 1, **characterised in that** the solubilisation is carried out at a rate of 100 to 300 g/l of white almond cake.

3. A method of obtaining an active principle with an immediate firming effect on the skin according to claim 1 or 2, **characterised in that** the white almond cake is a cake of Prunus Amygdalus Dulcis almonds.

4. A method of obtaining an active principle with an immediate firming effect on the skin according to any one of the preceding claims, **characterised in that** the polymerisation initiator comprises at least 1% of a multifunctional compound such as an acid polychloride, an acid polyanhydride, a polyisocynate or a polythioisocyanate.

5. A method of obtaining an active principle with an immediate firming effect on the skin according to any one of the preceding claims, **characterised in that** the polymerised protein fraction has a molecular mass of more than 600 kdaltons.

6. An active principle obtained by implementing the method defined according to any one of claims 1 to 5, **characterised in that** it presents the following parameters:
- dry matter level above 50 g/l, preferably between 50 and 500 g/l and more particularly limited to the range 50/250 g/l,
- pH between 2.0 and 10.0, in particular 4.0 and 10.0, more specifically 7.0 and 9.0,
- protein level compared with dry matter greater than 70%,
- distribution of molecular weights 50/50 of
* proteins of dimer form with a molecular weight of between 30 and 51 kdaltons
* proteins with very high molecular weight above 600 kdaltons.

## Patentansprüche

1. Verfahren zur Gewinnung eines Wirkstoffes mit sofortiger straffender Wirkung auf die Haut, **dadurch gekennzeichnet, dass** es die folgenden Schritte umfasst:
- Solubilisieren eines Presskuchens aus weißen Mandeln in wässriger Phase, so dass der Mandelgehalt 50 bis 500 g/l beträgt,
- Durchführen einer gemäßigten enzymatischen Hydrolyse bei saurem pH und einer Temperatur im Bereich von 40 bis 80 °C,
- Trennen der löslichen und unlöslichen Phasen und Inaktivieren des Mediums zur Unterbrechung der enzymatischen Reaktion,
- Polymerisieren der zurückgehaltenen Proteine durch Zusetzen eines Polymerisationsinitiators, und
- Abtrennen der polymerisierten Proteine.

2. Verfahren zur Gewinnung eines Wirkstoffes mit sofortiger straffender Wirkung auf die Haut nach Anspruch 1, **dadurch gekennzeichnet, dass** das Solubilisieren des Presskuchens aus weißen Mandeln so durchgeführt wird, dass der Mandelgehalt 100 bis 300 g/l beträgt.

3. Verfahren zur Gewinnung eines Wirkstoffes mit sofortiger straffender Wirkung auf die Haut nach Anspruch 1 oder 2, **dadurch gekennzeichnet, dass** der Presskuchen aus weißen Mandeln ein Presskuchen aus Prunus Amygdalus Dulcis Mandeln ist.

4. Verfahren zur Gewinnung eines Wirkstoffes mit sofortiger straffender Wirkung auf die Haut nach einem der vorangegangenen Ansprüche, **dadurch gekennzeichnet, dass** der Polymerisationsinitiator mindestens 1 % einer multifunktionellen Verbindung, beispielweise eines Polychlorids einer Säure, eines Polyanhydrids einer Säure, eines Polyisocyanats, eines Polythioisocyanats umfasst.

5. Verfahren zur Gewinnung eines Wirkstoffes mit sofortiger straffender Wirkung auf die Haut nach einem der vorangegangenen Ansprüche, **dadurch gekennzeichnet, dass** der Anteil an polymerisierten Proteinen ein Molekulargewicht von mehr als 600 kDalton aufweist.

6. Wirkstoff, wie er durch die Durchführung des nach einem der Ansprüche 1 bis 5 definierten Verfahrens gewonnen wurde, **dadurch gekennzeichnet, dass** er die folgenden Parameter aufweist:
- Trockenmassegehalt von mehr als 50 g/l, vorzugsweise von zwischen 50 und 500 g/l und insbesondere auf den Bereich von 50/250 g/l beschränkt,
- pH von zwischen 2,0 und 10,0, insbesondere von zwischen 4,0 und 10,0, noch spezifischer von zwischen 7,0 und 9,0.
- Proteingehalt von mehr als 70 % bezogen auf die Trockenmasse.
- Verteilung der Molekulargewichte 50/50 von
* Proteinen in dimerer Form mit einem Molekulargewicht von zwischen 30 und 51 kDalton
* Proteinen mit einem sehr hohen Molekulargewicht von mehr als 600 kDalton.
